# EUROPEAN PATENT APPLICATION

(11) **EP 1 752 173 A1**
(43) Date of publication of application: **14.02.2007**
(21) Application number: 05719403.7
(22) Date of filing: 23.02.2005
(51) Int. Cl.: A61M 5/158, A61M 5/32, A61M 25/00

(54) **HOLLOW NEEDLE AND INDWELLING NEEDLE USING THE HOLLOW NEEDLE**

(30) Priority: 31.05.2004 JP 2004160760
(71) Applicant: Institute of Whole Body Metabolism, Shiroi-shi, Chiba 270-1407 (JP)
(72) Inventor: SHIGEMATSU, Akiyo, 2701436 (JP); YUI, Jyoji, 2701335 (JP); HAMAI, Yuko, 2701326 (JP)
(74) Representative: Siegert, Georg
(86) International application number: PCT/JP2005/002848
(87) International publication number: WO 2005/115505

(57) **Abstract**

[ PROBLEMS ] Anon tissue-destructive hollow needle not damaging the tissue of a needle inserting portion and an indwelling needle using the hollow needle. [ MEANS FOR SOLVING PROBLEMS ] A hollow needle is made from ceramic, has an outer diameter of 1 - 5 mm, and a wall thickness of 0.6 - 1.8 mm, and gives less damage to the tissue of a needle inserting portion. Worth noting is that the ceramic is zirconium and its oxide. The non tissue-destructive hollow needle can be used as a indwelling needle. The indwelling needle is constructed by inserting a resin straight fine needle, with chemical resistant and corrosion resistant properties, into the ceramic hollow needle, inserting the hollow needle, installed on the straight fine needle, into a hollow needle outer tube, and providing a connection tube, adapted for connection to an external extension tube, on the hollow needle outer tube. The connection tube is connected to the external extension tube through a connection ring. A fluorocarbon resin is suitably used as the chemical resistant, corrosion resistant resin.

## Description

### Technical Field

The present invention is related to a hollow needle not damaging the tissue of a needle inserting position and indwelling needle using the hollow needle.

### Background Art

There are such needles as a biopsy needle for sampling some body tissue, an indwelling needle used for the blood collection and delivery, and an injection needle used for the drug injection, widely used in the field of medicine. Almost all of the biopsy needle, indwelling needle, and injection needle are made by stainless steel. More specifically, the biopsy needle, the indwelling needle, and the injection needle are made by a hard and stain-free material including the stainless steel as the predominant constituent. However, since the needle made by the stainless steel is soft to be magnetized and is conductive, arising the problem of causing the sampled tissue to become non-vital. In addition, the tissue portion punctured by the needle incurs some extent of damage.

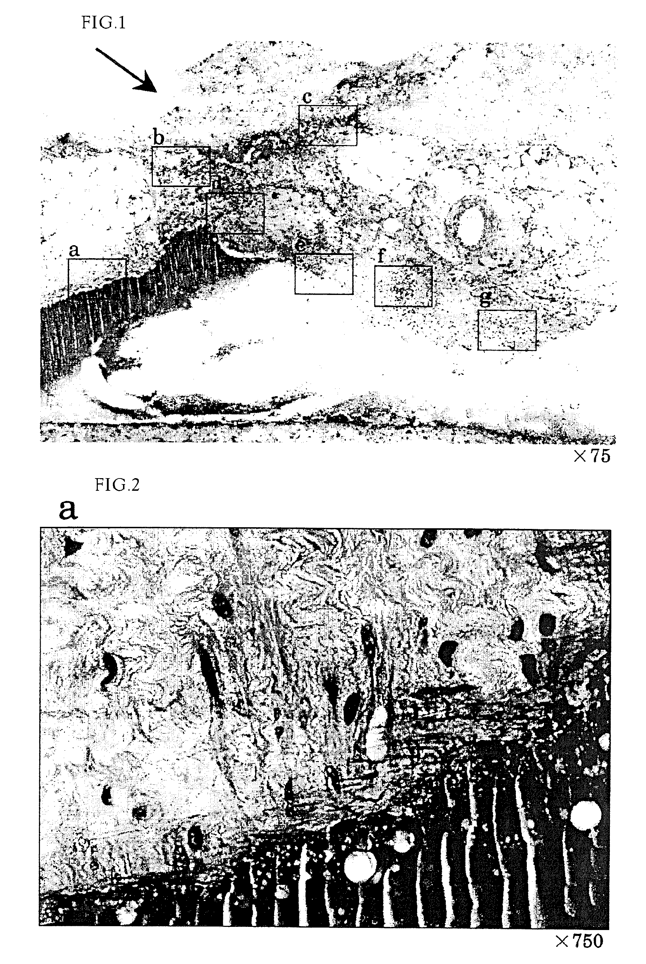

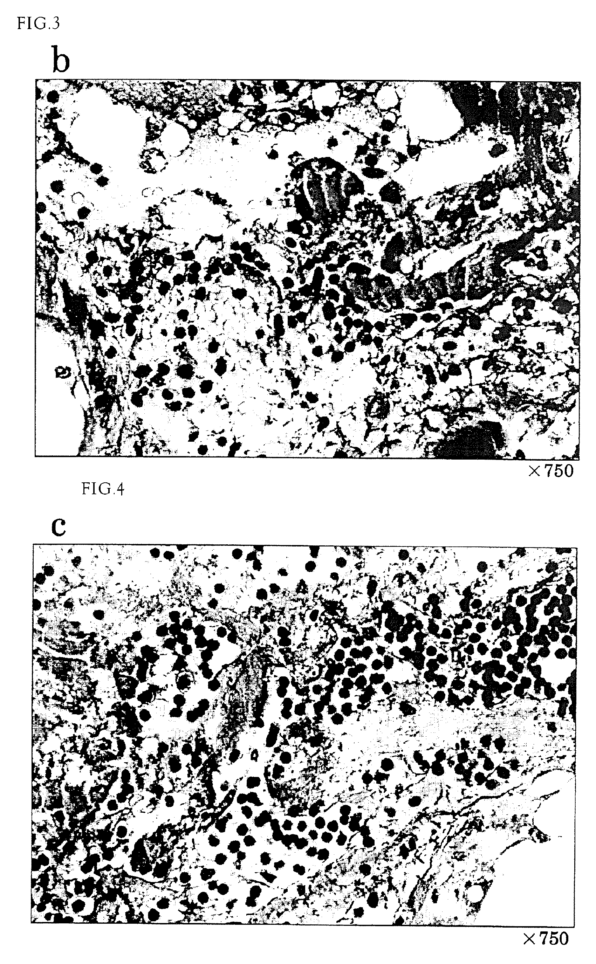

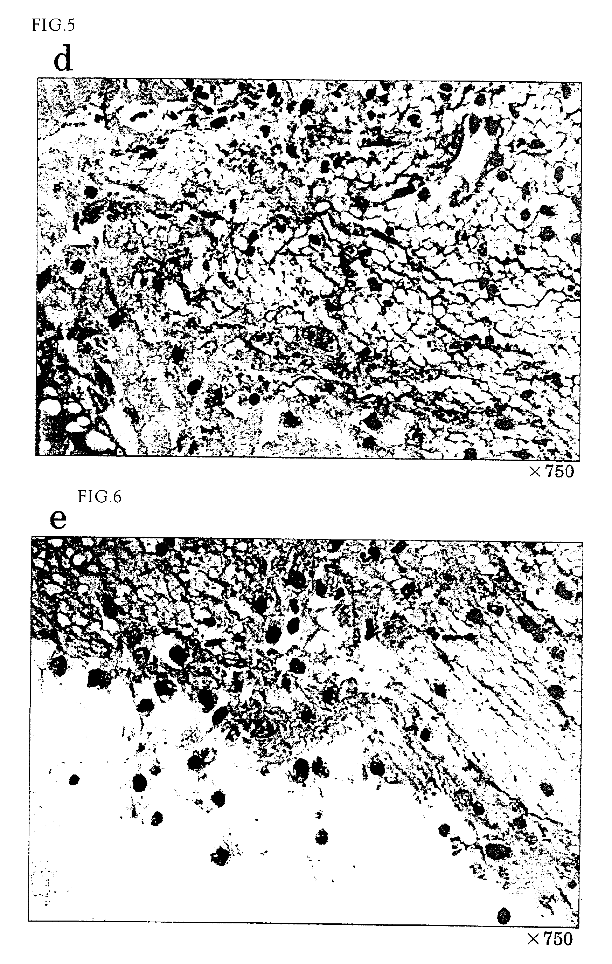

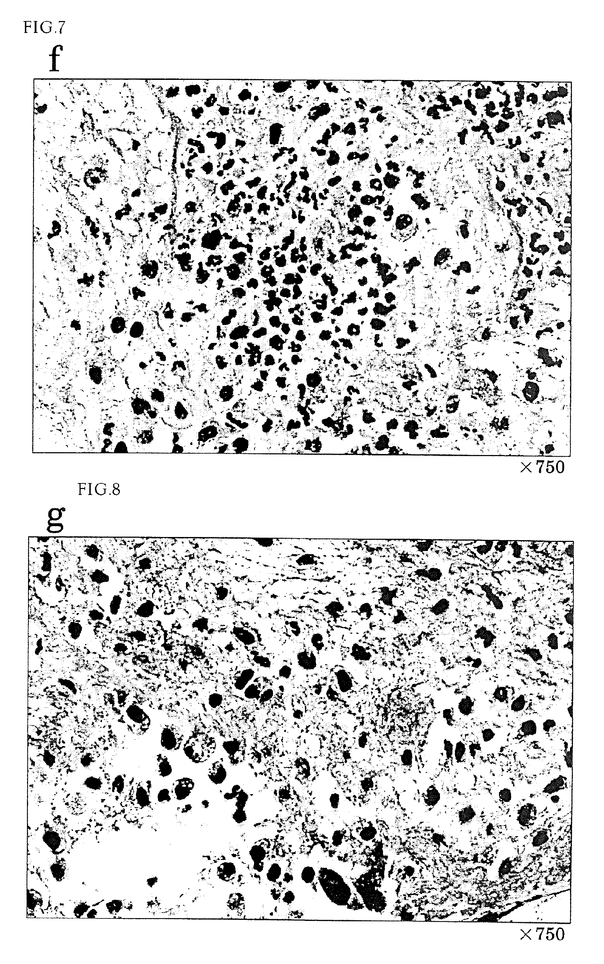

As will be described in greater details in the preferred embodiments herein below, at the time of using the biopsy needle, the indwelling needle, and the injection needle made by a stainless steel, the tissue of the puncture site will be damaged. Now an example of tissue damage will be described in which an indwelling needle made by a stainless steel is inserted and drawn 2 minutes after the punctuation at the inferior part of the inferior vena cava of a rat, following by the hemostasis operation. Fig. 1 shows an inferior vena cava of a rat. White blank space occupying approximately one third of the bottom of Fig. 1 is the lumen of the vena cava. Fig. 2 to Fig. 8 show the partial enlargement of sites labeled by a, b, c, d, e, f, and g in Fig. 1. Fig. 3 and Fig. 4 indicate the superior wall of the vena cava, the portion as seen as bubbles indicates the presence of hemostatic agent. Fig. 3 and Fig. 4 presents some basophiles among leucocytes, which sensitively react to some allergen including histamine to ultimately provide the inflammation. Fig. 7 indicates reactive neutrophiles reacting to the interfused fluid of the intra-cell component, caused by the tissue injury, which actively indicate the phagocytosis. Fig. 6 and Fig. 8 indicate some eosinophiles, which contain some components involving the inflammatory process such as histamine. The eosinophiles are evoked and proliferated by the immune response. Fig. 2 shows a clot formed by the reaction between the infiltrate fluid and blood in the bloodstream in response to the damage of the inner wall of the vein lumen. All of these tissue images are three days after the punctuation, which indicates that the inflammation is so severe that the recovery or restoration is not yet prognostic.

In JP-A-H3-193061 publication (patent reference 1), there is described a punctuation needle made of a ceramic, as nonferrous hollow needle, which does not brake in use but may be easily processed after the use, because the metal needles are not able to be post-processed such as shredding. However the punctuation needle disclosed in JP-A-H3-193061 is fragile and may not be served actually as an injection needle or an indwelling needle. In JP-A-H7-16297 (patent reference 2), there is disclosed a medical needle made by a ceramic. The needle disclosed in the above publication is stated that it has the effect that it can be safely and harmlessly processed by burning. However this needle is also too fragile to practically serve actually as the injection needle or the indwelling needle.

In JP-A-2003-334199 publication (patent reference 3), there is disclosed that a ceramic indwelling plug may be used in place of a plastic indwelling plug used hitherto in the hemodialysis. It is stated that the ceramic indwelling plug has the effect not to induce the downgrowth (rejection reaction) and allergic reaction developed when using the plastic indwelling plug. However, the indwelling plug is not a hollow needle for use in the injection needle or the indwelling needle, which does not alleviate the tissue injury at the punctured site, neither.

In JP-A-2003-305121 (patent reference 4) and JP-A-2003-210579 (patent reference 5), there is disclosed a hollow needle having a non-conductive ceramic film formed on a metallic hollow needle. It is stated that this needle may not be broken in use has the effect that it does not affect to the collected tissue sample and the surrounding cells around the punctured site of the lesion. However, because the hollow needle having a ceramic film formed on a metallic hollow needle has its tip not covered by the ceramic, there remains the problem that the tissue at the punctuation site will be damaged.

The inventors of the present invention invented in the past an injection needle or an indwelling needle for use in the biopsy having a hollow fine tube formed from a nonferrous material and covered by a extremely hard, non-conductive, non-magnetizable material in order to prevent the deactivation of tissue sample collected, and claimed a patent protection accordingly (see JP-A-2002-85413 (patent reference 6)). This patent application has the object to mainly provide a biopsy needle for collecting the tissue located in a deep location in the body, and has the problem that the manufacturing process of the hollow needle may become complex because of covering a hollow fine tube formed from a nonferrous material with an extremely hard, non-conductive, non-magnetizable material.

As can be appreciated from the foregoing description, in the medical field, hollow needles made by a metal such as stainless steel have been widely used, however such needles may have the problems of disposition after use, the deactivation of collected sample tissue, and the like. To remedy the problems, the hollow needle made by a ceramic or the hollow needle coated by a ceramic film have been proposed. However, the hollow needle made by a ceramic material has the problem of fragility, while the hollow needle covered by a ceramic film has the problem of complex manufacturing process. There is still unknown a hollow needle which may prevent the damage of tissue at the punctured site when punctuating with the hollow needle. Although in the patent references 4 and 5, it is suggested that the hollow needle may affect to the cells around the punctuation site in the lesion, a hollow needle which may alleviate the damage of tissue at the punctuation site is still unknown as have been described above.
patent reference 1: JP-A-H3-193061
patent reference 2: JP-A-H7-16297
patent reference 3: JP-A-2003-334199
patent reference 4: JP-A-2003-305121
patent reference 5: JP-A-2003-210579
patent reference 6: JP-A-2002-85413

### Summary of the invention

### Problem to be solve by the invention

As have been stated above, the needle most numerous and frequently used in the medical field is the hollow needle including the injection needle, the biopsy needle, and the indwelling needle, and the almost all of which are made from a stainless steel. The needle made from a stainless steel is magnetizable and conductive, so that it may develop a damage potential in the living body, inducing the cell death approximately hundreds micrometers around the needle punctuation point. The inventors of the present invention have recognized the effect that the hollow needle covered by a non-magnetizable material (ceramic) prevents the deactivation of the collected tissue sample. This suggests that the needle made by a ceramic has basically an effect of preventing the deactivation of the collected tissue sample. The present invention has been made in view of the above circumstances and has an object to provide a hollow needle which minimizes the damage of tissue at the hollow needle punctuation site when punctuating the hollow needle into the tissue, as well as to provide an indwelling needle using the hollow needle which minimizes the damage of the tissue.

### Means for solving the problem

In summary the present invention provides a hollow needle made by a ceramic material and an indwelling needle using the hollow needle. More specifically, the hollow needle is made by a ceramic material and has an outer diameter of 1 mm to 5 mm, which is unlikely to damage the tissue at the needle punctuation site. It is preferable that the needle has a wall thickness of 0.3 mm to 1.8 mm. The ceramic material is preferably fine powder of zirconium or zirconium oxide. In addition, the indwelling needle uses the tissue-undestructive hollow needle, inserted into a resin tube of chemical resistant and corrosion resistant properties. The indwelling needle is made by inserting a ceramic hollow needle into a resin straight fine tube having the chemical resistant and corrosion resistant properties, installing the hollow needle inserted into the resin straight fine tube of chemical resistant, corrosion resistant property into a hollow needle outer tube, and providing a connection tube for connection to the external extension tube on the hollow needle outer tube. A fluorocarbon resin is suitably used as the chemical resistant, corrosion resistant resin.

The hollow needle made by a ceramic in accordance with the present invention may be made more specifically by providing a heat resistant wire as the center axis, coating a uniform thickness on the outer side thereof with ceramic fine powder paste of zirconium or its oxide and then baking, then removing said wire after baking. The non punctuating tip of the hollow needle will be inserted into a thick straight fine tube made of chemical resistant, corrosion resistant resin. More specifically, the non punctuating tip of the hollow needle is inserted to an end of a thick straight fine tube made of chemical resistant, corrosion resistant resin and secured to the straight fine tube. A head having non-woven cotton may be provided to the other end of the straight fine tube. The non-woven cotton may be conveniently used in the indwelling needle for confirming that the blood is flowing from within the hollow needle through the straight fine tube. Next, the hollow needle inserted to the end of a thick straight fine tube made of corrosion resistant resin is then inserted into a hollow needle outer tube made of chemical resistant, corrosion resistant resin. The hollow needle insertion section of the hollow needle outer tube is consisted of a thick center tube made of chemical resistant, corrosion resistant resin, and the thick insertion section of the outer tube into which the thick straight fine tube made of chemical resistant, corrosion resistant resin installed to the non punctuation end of the hollow needle is inserted is made of a thin outer tube made of chemical resistant, corrosion resistant resin. Further, a flexible connection tube is installed to the hollow needle outer tube at the position of insertion of the outer tube for connection to the external extension tube. There may be provided a needle ring on the hollow needle end of the connection tube, and a connection ring on the other end thereof. The external extension tube may be an extension tube for a dialyzer or an extension tube for drug infusion. In practice the external extension tube is connected at the position of the connection ring. A clip may be provided to the connection tube in order to temporarily stop the flow of liquid in the connection tube. The clip is used to temporarily stop the flow of blood or drug to perform any necessary operation when required.

The hollow needle outer tube may be obtained by inserting a mold in a thin tube made of chemical resistant, corrosion resistant resin, then heat processing the entire member to shrink the size. The mold may be made of a piece of metal, woodwork, or plastic. A heat resistant mold is preferably used for enduring the heat process. The mold is in the shape of a cylinder, and approximately half of the total length is tapered.

As chemical resistant, corrosion resistant resin, there are suitably used resins such as polyester resin, polyvinyl chloride resin, polyethylene resin, polyolefin resin, polyamide resin, polyurethane resin, silicone resin, and a fluorocarbon resin is preferably used. As the material for forming the connection tube there are suitably used resins as similar to the chemical resistant, corrosion resistant resins as listed above, such as polyester resin, polyvinyl chloride resin, polyethylene resin, polyolefin resin, polyamide resin, polyurethane resin, silicone resin, and the silicone resin is preferably used.

The frequency of long time blood collection, and blood and drug infusion is increasing due to the increase of patients of chronic renal diseases and elder patients. Since a hollow needle made of a stainless steel is used for the prolonged blood collection and infusion, the tissue of such patients consequently will be damaged at the time of punctuation by the hollow needle. Because of such circumstances, a hollow needle used for the injection needle and the indwelling needle which may impair less tissue at the needle punctuation site is claimed in the medical field.

The hollow needle in accordance with the present invention is basically made of a ceramic. While the hollow needle made of a stainless steel commonly used in the practice may damage the tissue at the needle punctuation site, the hollow needle made by a ceramic in accordance with the present invention minimizes the damage of tissue at the needle punctuation site. There is a considerable difference of the extent of damage of tissue at the needle punctuation site between a stainless steel needle and a ceramic needle, as will be described more specifically in the preferred embodiments herein below.

This is the first time that the inventors of the present invention have discovered that, when using a stainless steel hollow needle, the tissue at the needle punctuation site may be damaged, while on the other hand the damage of tissue is considerably alleviated when using a ceramic hollow needle.

As disclosed in JP-A-H7-16297 document (patent reference 2), a needle made from a ceramic for medical use has a problem of fragility. From the viewpoint of the fragility of a ceramic needle, it is preferable for the hollow needle to have a constant wall thickness. More specifically, the outer diameter of the hollow needle is preferably more than 20G (gauge) or larger than 1 mm, Furthermore there is almost no breakage when 14G (outer diameter 2.11 mm) is used. Since the hollow needle is served for punctuating a blood vessel, its outer diameter is affected by the diameter of blood vessel. A hollow needle of suitable outer diameter should be selected in accordance with the punctuating position and the usage. With respect to the wall thickness, a thicker wall may be less breakable, however a thick wall may result in a narrower lumen diameter, and consequently may cause less amount of inflow blood rate per unit time, therefore the wall thickness may be preferably more than 0.3 mm and less than 1.8 mm. The wall thickness surely is a function of the outer diameter. In general, a larger outer diameter the needle has, a thicker wall thickness it has accordingly. In a hollow needle made from zirconium, the influence to the proteins in the blood before and after flowing therethrough is considerably minimized when compared with a hollow needle made from a stainless steel having such characteristics as conductive and magnetizable.

A hollow needle made from a ceramic may be obtained by burning a nonferrous material. Some examples of nonferrous material, include aluminum, titanium, silicone, nickel, tungsten, zirconium, and the oxides thereof. Aluminum, titanium, silicone, nickel, tungsten, and zirconium are nonferrous metal, which become oxide when burning, suitable to form a ceramic needle. The hollow needle in accordance with the present invention may be suitably made from zirconium and zirconium oxide in particular and burned.

The hollow needle made from a ceramic may be manufactured for example as stated below. A straight carbon wire, or a wire of a material which evaporates or melts by the oxidation at the temperature of more than 1000 degrees Celsius is used as the core. The core is wrapped by a very thin sheet of the paste of the very fine powder of nonferrous material, having the thickness of 0.5 mm to 2 mm, then air-dried. If a backside of the wire is applied with a oleophilic cream (for example a mixture of Vaseline and liquid paraffin in a ratio of 95:5) immediately prior to covering the wire with the paste of the very fine powder of nonferrous material, the wire can be withdrawn from the clad film made of the nonferrous material after air-dry. In case in which the clad film of the nonferrous material significantly shrinks during burning, the material can be alternatively burned with the wire, and the wire can be evaporated at the temperature of more than 1000 degrees Celsius. The tip of thus burned hollow tube may be abraded with a diamond grindstone to obtain a hollow needle having the inclination angle of 10 to 30 degrees.

Alternatively, the ceramic paste of for example zirconium very fine powder or of zirconium oxide very fine powder may be applied to the metallic mold of the hollow needle, air-dried and burned to obtain a hollow needle made of the ceramic. In this case the mold used as the medial core can be made of cast plaster. The withdrawal of the mold from the ceramic hollow needle may become difficult if the shrinking rate of the mold at the time of burning is less than the shrinking rate of the ceramic during burning, the mold is preferably made of a material which decays or melts at a lower temperature such as the plaster or aluminum. When burning the very fine powder of the nonferrous material, the material becomes oxide during burning, and yields the ceramic. A carbon core can be used as the mold. The tip angle of the hollow needle may be acute of approximately 10 to 30 degrees in accordance with the application.

The hollow needle for the injection needle or the indwelling needle in accordance with the present invention can be served as the needle for long time sampling or long time infusion. The hollow needle used as an injection needle or indwelling needle is made from a ceramic, and has an outer diameter of 1 to 5 mm. The wall thickness of the hollow needle is preferably in the range of 0.3 to 1.8 mm. The wall thickness may be selected suitably in accordance with the outer diameter.

An indwelling needle using the hollow needle which may affect less damage to the tissue at the position of punctuation will be described in greater details. The non punctuating end of the hollow needle made of a ceramic is inserted into a straight fine tube of a thick wall made of a chemical resistant, corrosion resistant resin. More specifically, the non punctuating end of the hollow needle is inserted into the straight fine tube, then the hollow needle is secured to the straight fine tube. At the other end of the straight fine tube a head having a Millipore filter section consisted of non-woven cotton is placed. Then, the hollow needle installed to one end of the straight fine tube of the chemical resistant, corrosion resistant resin is inserted into a hollow needle outer tube. The section of the hollow needle outer tube into which the hollow needle is inserted is consisted of a thicker tube (center tube) having a narrower lumen diameter into which the hollow needle can be inserted, and the section of the hollow needle outer tube into which the straight fine tube is inserted is consisted of a thinner tube (receiving tube) having a lumen diameter sufficient to insert the straight fine tube, made of a chemical resistant, corrosion resistant resin. The receiving tube section of the hollow needle outer tube is provided with a connection tube to the external extension tube for connecting to an extension tube of a dialyzer. The connection tube may be a flexible silicone tube for example. A clip can be provided to the connection tube. The indwelling needle may be constructed by inserting the thick straight fine tube of the chemical resistant, corrosion resistant resin installed to the non punctuating end of the ceramic hollow needle into the hollow needle outer tube. The connection tube is installed on the receiving tube of the hollow needle outer tube. The injection fluid or blood flows through the hollow needle outer tube. A clip can be provided to temporarily stop the flow of the injection fluid or blood. More specifically, the injection fluid or blood flowing through the receiving tube can be stopped by nipping the connection tube provided on the receiving tube with a clip. Some typical examples of the chemical resistant, corrosion resistant resins include polyester resin, polyvinyl chloride resin, polyethylene resin, polyolefin resin, polyamide resin, polyurethane resin, and silicone resin, in particular the fluorocarbon resin is suitably used.

### Effect of the invention

The hollow needle made of a ceramic in accordance with the present invention can be used for the injection needle and indwelling needle, which gives minimal damage of tissue at the needle punctuation site. In particular, the hollow needle in accordance with the present invention can be used for the long time blood sampling and the long time infusion. The hollow needle for the injection needle and indwelling needle in accordance with the present invention, which gives less damage of tissue at the punctuation side of the needle, may be served as the injection needle or indwelling needle for clinical blood sampling and blood injection in the wide aspect of clinical application. For example, in the dialysis, the blood vessel damage of a number of patents can be minimized.

The hollow needle in accordance with the present invention may be used for the blood sampling and injection, in particular for the long time blood sampling and the long time infusion. A typical example of long time blood sampling and the long time injection is the dialysis. In the dialysis conducted in Japan the dialysis is performed every other day, total of 360 punctuations of indwelling needle into the artery and vein for a patent necessary to dialyze per year. The total number of patents necessary to dialyze is said approximately 220,000. These patients undergo a dialysis with a column chromatographic method to remove the waste in the blood (such as the uric acid, urea, urobilin, and some salts) taken from their artery and to return the blood into the vein by adding some essential amino acids, hormones, vitamins, sugars, fatty acid glycerides and the like. In dialysis an amount of blood (approximately 4 to 5 litters) is desired to be dialyzed in a short period of time.

Among renal disease patents those requiring dialysis of blood regularly may have a choice of two options in general, and the most patients selects the hematogenous shunt method. The shunt method is consisted of increasing the blood flow by the anastomotic operation of an artery and a vein in the brachium, applying a tourniquet of rubber belt to the downstream of the vein to form a blood pool, and then puncturing with an indwelling needle of stainless steel (in accordance with the current practice) of 18G. After punctuation the stainless steel needle is immediately withdrawn and the extension tube is connected to the head of the indwelling needle to feed the blood to the connection tube of the dialyzer. Then when the processed blood is flew out from the outlet tube of the dialyzer, the blood is returned to the infusion site of the vein downstream to the tourniquet on the brachium, through another stainless steel indwelling needle of 18G already punctured with the stainless steel needle removed from the indwelling needle, the return tube is connected to the head of the indwelling needle.

In practice, the dialysis requires the connection operation of an artery and a vein to an artificial external tube. The dialysis, needless to say, may not be operated without some damage of the blood vessel. At this time, a thicker indwelling needle is preferable due to the capability of collecting much blood. There are not rare cases of abnormal stimulation due to the contact of blood cells and proteins (albumin and globulins) with the artificial tube. In most patients the dialysis for a long period of time (maybe lifelong in many cases) causes amyloidosis, which supervenes sequela. In order to elude such side effect, the non tissue damaging needle for the injection needle or indwelling needle in accordance with the present invention is very practicable.

The indwelling needle in accordance with the present invention has an effect of minimizing the injury of the blood vessel endothelial cell layer, and significant recovery. This is because the ceramic indwelling needle is not intervened by heat, electricity, and magnetic, only the chemical resistant, corrosion resistant resin is in contact with the living tissue after the single moment of punctuation, the membrane damage of cell membrane of the flow-in, flow-out blood cells, platelets, and granulocytes. In practice, from the two-dimensional image analysis using [2-14C] thymidine, as shown in the preferred embodiments of the present invention, the injury of the blood vessel endothelial cell membrane caused by the punctuation of a ceramic indwelling needle is quickly recovered by the stem cell based on the neogenesis of endothelial cells, and it is confirmed that the genesis of muscular stem cells served for the recovery of ring-shaped muscle is activated within 72 hours from the injury.

### Best mode for carrying out the invention

Some preferred embodiments of the present invention will be described in greater details herein below. The present invention is not limited thereto.

### [first embodiment]

### Manufacturing a hollow needle (1)

The paste of zirconium oxide very fine powder was applied to a mold, dried and burned at 1, 300 degrees Celsius for an hour to obtain a hollow needle. The mold used as the core was made from the plaster. In this manner a hollow needle having the outer diameter of 1.8 mm, wall thickness of 0.7 mm, length of 40 mm was obtained. The mold might not be easily withdrawn from the zirconium oxide hollow needle if the shrinking rate of the mold during burning is less than the shrinking rate of zirconium oxide during burning, it is preferable that the mold is made from the plaster or aluminum, which decays or melts at a lower temperature. It is to be noted here that the zirconium fine powder is also applicable. The zirconium fine powder transforms to zirconium oxide during burning. Also, the carbon core is also usable as the mold. The tip angle of the hollow needle may be acute, approximately 10 to 30 degrees according to the application. In a similar manner, a ceramic hollow needle can be obtained from any of nonferrous materials other than the zirconium and its oxide, such as aluminum, titanium, silicone, nickel, tungsten, and the oxide thereof.

### [second embodiment]

### Manufacturing a hollow needle (2)

A hard solid sheet of zirconia (zirconium oxide) having a uniform thickness of 2.5 mm was formed by kneading zirconia fine powder with water. The thickness of sheet is preferably three times the thickness of final hollow needle product. The sheet of uniform thickness was cut into a rectangular piece. A wire of the diameter of 3 mm with mineral oil applied was used as the core, the rectangular sheet was wrapped therearound firmly so as to engulf the core to form a cylinder of zirconia. The diameter of the wire is preferably 1.5 times of the outer diameter of the hollow needle final product. The cylinder made of zirconia was air-dried in a dryer room. A straight carborundum (having a diameter slightly smaller than the lumen diameter) was used for the core so as not to lose the linearity of the cylinder to rotate slowly while burning in an electric furnace at 1,300 degrees Celsius for 1 hour 30 minutes. The cylinder thus completed was cooled, picked up after cooling and then the wire was withdrawn to obtain a hollow needle. The needle was finished by grinding with a diamond cutter at an acute angle of approximately 20 degrees. The hollow needle thus obtained has its outer diameter of 2 mm, wall thickness of 0.8 mm, and length of 4.5 mm. The needle thus finished had a longitudinal strength larger than that of a ferrous needle. The needle was not easily broken when applying a force at 90 degrees to the longitudinal axis. The tip angle of the hollow needle may be appropriately acute as 10 to 30 degrees according to the application. The physical property of burned zirconia was very rigid, non-conductive, non-magnetizable, which has been discovered that the biologic reaction is minimized after punctuation to a living body, and different from the stainless steel needles, the injury caused by the punctuation is rapidly recovered, from the preferred embodiments as will be described later. Zirconium fine powder may be used instead. This is because the zirconium fine powder transforms to zirconia during burning. In a similar manner, a ceramic hollow needle can be obtained from such nonferrous materials as aluminum, titanium, silicone, nickel, tungsten and the oxide thereof, other than the zirconium and zirconia.

### [third embodiment]

### Influence of zirconia hollow needle to the tissue

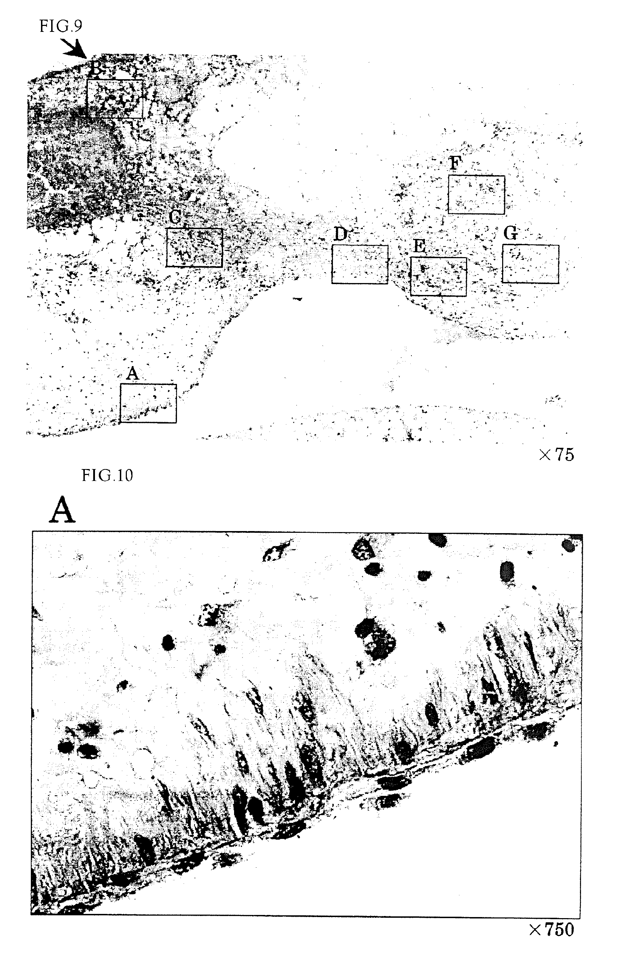

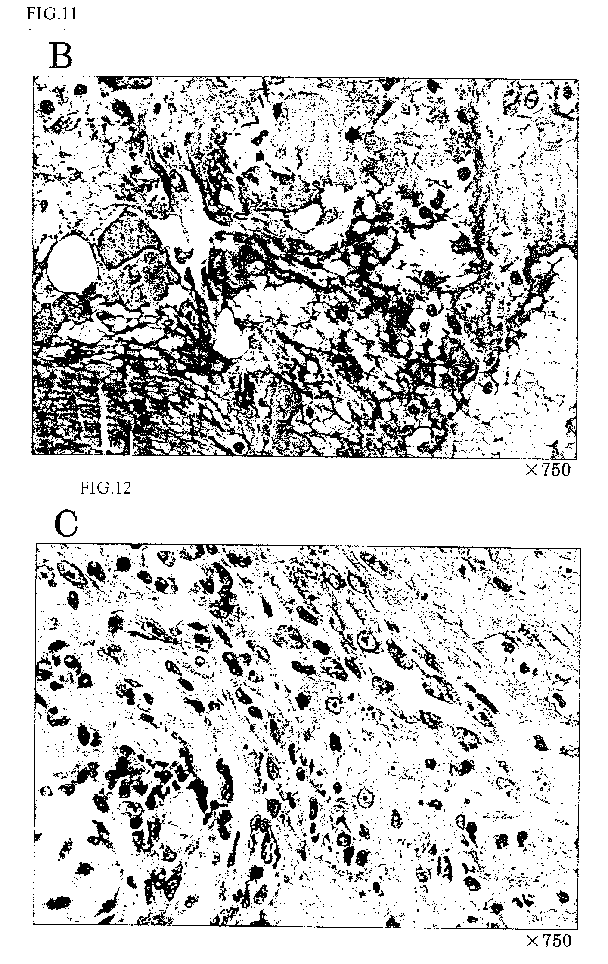

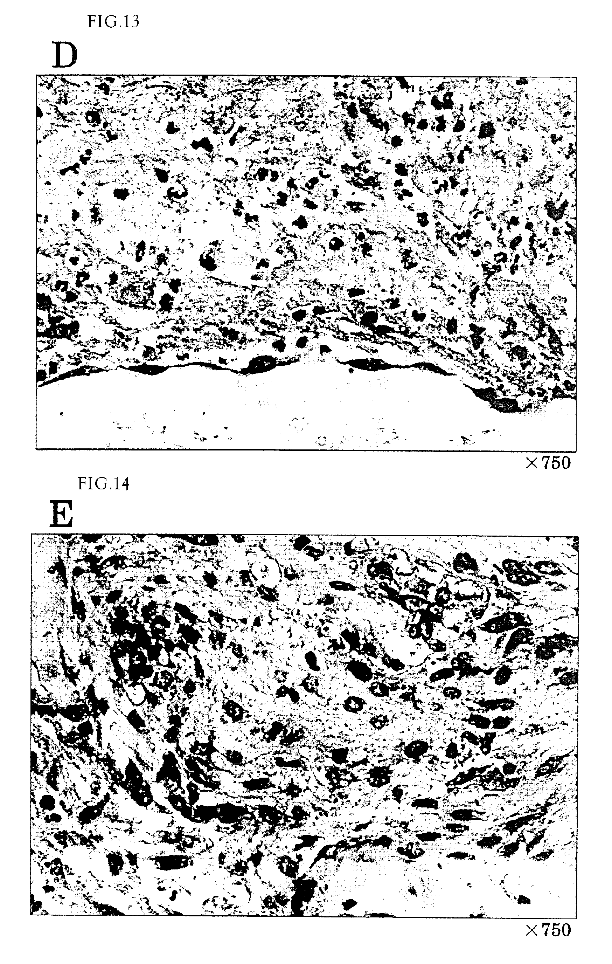

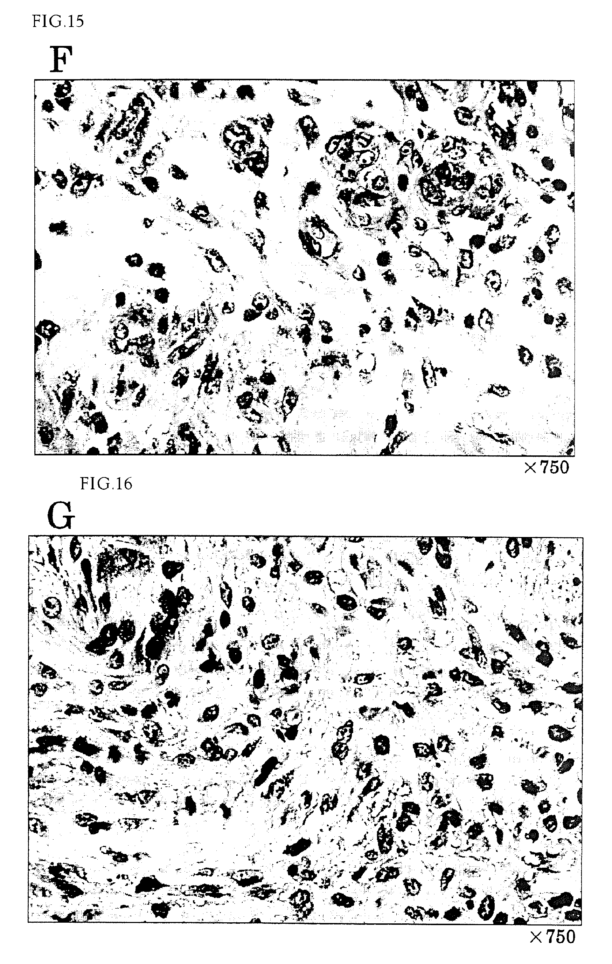

The inferior vena cava of a rat was punctured with the zirconia hollow needle made in accordance with the second preferred embodiment, the tissue three days after the punctuation was observed by a light microscope. The inferior vena cava of a rat was punctured with the zirconia hollow needle, and the needle was withdrawn two minutes after the punctuation and hemostasis was done thereafter. The tissue seen by a microscope at that time is shown in Fig. 9. Some sections of Fig. 9 labeled by A, B, C, D, E, F, and G were enlarged in Fig. 10 to Fig. 16. In Fig. 10 cell layers (endothelial layers) apposed from left to right of normal blood vessel inwall layers are clearly shown. After punctuation, as shown in Fig. 13, in contrast, a layer of the inwall layers is already restored three days after the punctuation. Also as shown in Fig. 12, Fig. 14, Fig. 15, and Fig. 16, purple tissue areas indicates the proliferation active for the restoration of muscle layer (middle layer as shown in Fig. 14, outer layer as shown in Fig. 3, Fig. 15, and Fig. 16). However, as shown i Fig. 13, part of muscle in the middle layer is recognized as restored, but the phagocytosis of neutrophiles is observed. The outside of the outwall of inferior vena cava is the portion in contact with the abdominal cavity, in which the phagocytosis of basophiles for cleaning after bleeding was observed. Approximately 80 to 90 % recovery of the blood vessel wall three days after the punctuation was observed in any specimen. This indicates the excellent proof of non tissue damage by the zirconia hollow needle.

### [fourth embodiment]

### Influence of stainless steel hollow needle to the tissue

The inferior part of the inferior vena cava of a rat was punctured with a stainless steel hollow needle, the needle was drawn two minutes after the punctuation and hemostatic operation was conducted. Fig. 1 shows the inferior vena cava. The white blank space at the lower third of Fig. 1 is the lumen of the vein. Then the sections labeled by a, b, c, d, e, f, and g, in Fig. 1 was enlarged and shown as Fig. 2 to Fig. 8. Fig. 3 and Fig. 4 indicates the superior wall of the vena cava, the portion seen as bubbles indicates the presence of hemostatic agent. Fig. 3 and Fig. 4 presents some basophiles among leucocytes, which sensitively react to some allergen including histamine to ultimately provide the inflammation. Fig. 7 indicates reactive neutrophiles reacting to the interfused fluid of the intra-cell component, caused by the tissue injury, which actively indicate the phagocytosis. Fig. 6 and Fig. 8 indicate some eosinophiles, which contain some components involving the inflammatory process such as histamine. The eosinophiles are evoked and proliferated by the immune response. Fig. 2 shows a clot formed by the reaction between the infiltrate fluid and blood in the bloodstream in response to the damage of the inner wall of the vein lumen. All of these tissue images are three days after the punctuation, which indicates that the inflammation is so severe that the recovery or restoration is not yet prognostic.

### [fifth embodiment]

### Preparation of indwelling needle

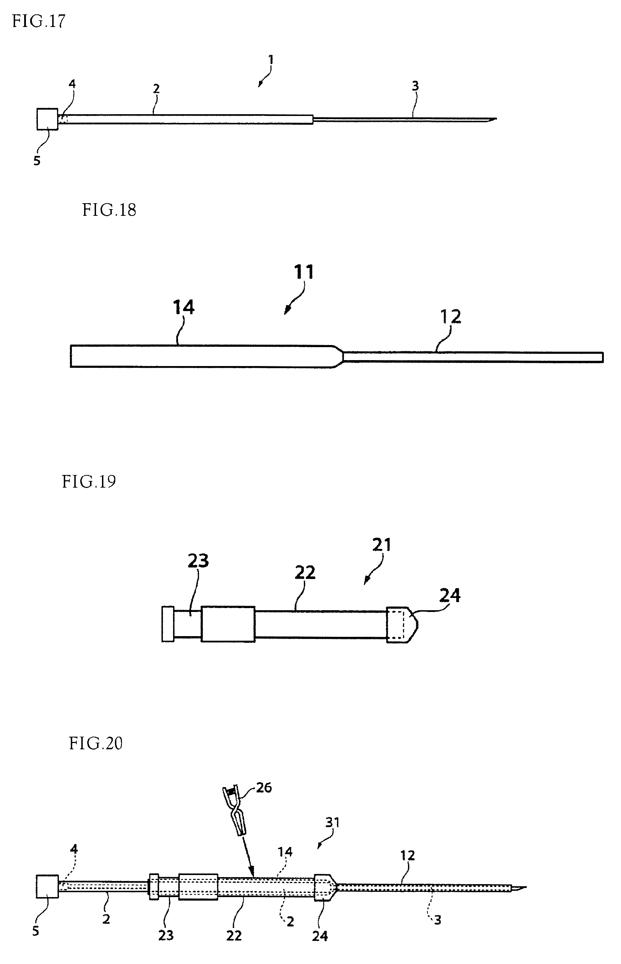

The arrangement of the indwelling needle 31 in accordance with the present invention is shown in Fig. 20, and members constituting the indwelling needle 31 are shown respectively, more specifically the hollow needle 1 with the straight fine tube is shown in Fig. 17, the hollow needle outer tube 11 is shown in Fig. 18, and the connection tube 21 is shown in Fig. 19. Basically the indwelling needle 31 is constituted of the hollow needle 1 with a straight fine tube as shown in Fig. 17, in which the non punctuating end of the hollow needle 3 is inserted into an end of the straight fine tube 2, and the hollow needle 3 is secured to the straight fine tube 2. A head 5 having a Millipore filter section comprised of non-woven cotton is provided at the other end of the straight fine tube 2. The straight fine tube 2 is a thick fine tube of a fluorocarbon resin, which is a chemical resistant, corrosion resistant resin. The straight fine tube 2 used herein has a lumen diameter sufficient to insert the hollow needle 3. The hollow needle 3 is the hollow needle made of a ceramic, as stated above, having the outer diameter of 1 to 5 mm, wall thickness of 0.3 to 1.8 mm. The straight fine tube 2 may be made of any chemical resistant, corrosion resistant resin, a fluorocarbon resin (more specifically PFA resin) was used in the embodiment. The straight fine tube 2 may have the thickness sufficient to have the strength for supporting itself horizontally without bending when placing horizontally.

The hollow needle 1 with the straight fine tube having the hollow needle 3 installed into the straight fine tube 2 was then inserted into the hollow needle outer tube 11 (see Fig. 18). The hollow needle outer tube 11 is consisted of a center tube 12, which has a smaller diameter, and a receiving tube 14, which has a larger diameter. The center tube 12 and the receiving tube 14 may be made of any of chemical resistant, corrosion resistant resins, and in the preferred embodiment a fluorocarbon resin (more specifically, resin commercially available under the name of PFA resin) was used. The hollow needle outer tube 11 was made from a thinner tube of a fluorocarbon resin having a larger outer diameter, a wooden mold was inserted into the thin tube, then the tube was shrunk by heat processing entirely so as to obtain a hollow needle outer tube 11, with the thick fine center tube 12 integral to the thin receiving tube 14. The thickness of the thick fine center tube 12 was four times the thickness of the thin receiving tube 14. In general the thickness of the thick fine center tube 12 is preferably twice to seven times the thickness of the thin receiving tube 14. The hollow needle 3 of the hollow needle 1 with the straight fine tube is inserted into the full length of the center tube 12 section, to fit to the lumen of the center tube 12, while the straight fine tube 2 of the hollow needle 1 with the straight fine tube was inserted into the receiving tube 14. The length of the hollow needle was 55 mm, and only the tip of the hollow needle 3 was projecting from the center tube 12 of the hollow needle outer tube 11. The wood mold is in a cylinder form, half of the length had a smaller diameter. The hollow needle outer tube 11 obtained by heat processing was cut appropriately to a suitable length to fit to the length of the hollow needle. The hollow needle outer tube 11 may be made by forming the center tube 12 and the receiving tube 14 separately and bonding together. Preferably the center tube 12 and the receiving tube 14 are integratedly made, and in the preferred embodiment an integrated hollow needle outer tube was used.

A connection tube 21 was provided to connect the hollow needle outer tube 11 and an external extension tube (more specifically this is the extension tube extended from a dialyzer apparatus). The connection tube 21 is consisted of a main connection tube 22, a needle ring 24 of the needle side placed at one end of the main connection tube 22, and a connection ring 23 placed at the other end of the main connection tube 22. The main connection tube 22 may be formed from any flexible materials, and in the preferred embodiment the main connection tube 22 of silicone rubber is used. The hollow needle outer tube 11 is inserted into the connection tube 21, then the main connection tube 22 is placed on the receiving tube 14. At this time the needle ring 24 is inserted from the side of the center tube 12 after inserting the hollow needle outer tube 11 into the connection tube 21 and fitted to the main connection tube 22 at the position where the center tube 12 is in contact with the receiving tube 14. The connection ring 23 ensure the connection to the external extension tube at the connection position of the main connection tube 22 to the external extension tube (not shown in the figure). The connection ring 23 may be separately prepared, inserted to the main connection tube 22 to secure it, or alternatively may be integratedly formed in the form of extension of the main connection tube 22. In the preferred embodiment a separately prepared ring is inserted onto the connection tube to secure it.

The overview of an indwelling needle 31 prepared in accordance with the above procedure is shown in Fig. 20. The use of the indwelling needle 31 will be described in greater details herein below. The indwelling needle set as shown in Fig. 20 is punctured into a blood vessel from obliquely downward to upward to insert the ceramic tip of the hollow needle into the blood vessel. The blood in the blood vessel reaches up to the Millipore filter section 5 at the rear end of the hollow needle with the straight fine tube 1 to change the filter color of the Millipore filter section 5 from white to red. This indicates the blood fills the hollow needle outer tube 11 made by a ceramic. Then, the main connection tube 22 of the connection tube 21 is nipped with a clip 26. Then the end of the indwelling needle 31 is grasped by left hand fingers to secure it, while the main connection tube 22 is firmly grasped by right hand fingers to secure it and to slowly withdraw the hollow needle 3 made by a ceramic. After the hollow needle 3 is completely withdrawn, the hollow needle with the straight fine tube 1 is disposed, and the external extension tube is connected by left hand fingers to the connection ring 23 held by right hand fingers then turn it 90 degrees to firmly connect the external extension tube and the hollow needle outer tube 11, so that the indwelling needle 31 is connected to the dialyzer. Thereafter the blood flows through the dialyzer and through the return extension tube. The clip 26 is removed after the dialyzer and the indwelling needle 31 are securely connected.

### [sixth embodiment]

### Application of the indwelling needle

Instead of human renal disease patient, a male rat of SD strain of eight weeks old was applied with the indwelling needle in accordance with the present invention. The outer diameter of the hollow needle forming the indwelling needle was 17G (gauge); the inner diameter corresponded to the lumen diameter of the downstream of right kidney of the inferior vena cava of the rat. The examinee rat was administered with 200 microliter of Nembutal into the abdominal cavity to entirely narcotize, then the abdomen was dissected to expose the ventral aorta. The needle ring 24 of the indwelling needle body in accordance with the present invention (Fig. 20) was nipped by a thumb and an index finger, and the wrist and elbow were firmly secured. Said ventral aorta was punctured from obliquely downward to upward to insert the needle tip into the blood vessel. Then the blood flew through the indwelling needle cavity to the Millipore filter section 4 at the rear end of the indwelling needle to change the filter color from white to red. This indicates that the blood is filled in the zirconia ceramic needle. Then a clip 26 is applied to the center of the main connection tube 22 proximal to the needle ring 24, the rear end of the indwelling needle is held and secured by left hand fingers (the thumb and the index finger). The right hand fingers were moved to the connection ring 23 of the main connection tube 22 of the indwelling needle to the dialyzer extension tube, the ring was nipped and secured with fingers while the hollow needle with the straight fine tube held by left hand fingers was slowly withdrawn. After the needle was completely withdrawn, the hollow needle with the straight fine tube 1 was disposed, then the main connection tube 22 is held by the left hand fingers to fit to the connection ring 23 held by right hand fingers and turn 90 degrees to connect the hollow needle outer tube 11 with the external extension tube. This terminates the connection operation between the indwelling needle 31 and the dialyzer. Thereafter, once the clip was released, the blood flew through the dialyzer to the return extension tube to the vein. In general, the blood flow is required to stop at or near the distal end connection of the return extension tube. Another indwelling needle is connected to the inferior vena cava of the rat in a similar manner at that time to connect the indwelling needle connection tube with the extension tube for the vena cava, so that the blood of the rat flew through the dialyzer and circulated entirely.

### [seventh embodiment]

### Biochemistry analysis of the blood when using indwelling needle

After the sixth embodiment was completed, the surgery and the blood biochemical analysis of the rat was conducted. Assuming that the dialysis time of the blood of the rat is 30 minutes (20 times the metabolic speed of human body), that of human might be 600 minutes (10 hours). Then, two indwelling sheaths (the center tube of the hollow needle outer tube: the hollow needle had been removed) retained to the aorta and vena cava for 30 minutes were removed, the abdominal dissection was sutured, then the blood samples were collected according to the scheduled elapsed time to analyze the transition of biochemistric concentration in the blood. The results were shown in Table 1. From Table 1, it is shown that the biological damage to the living body caused by the indwelling needle in accordance with the present invention was minimal.

**Table 1**

| | |
|---|---|
| GOT | 40 - 63 (IU/L) |
| GPT | 21 - 48 (IU/L) |
| body temperature | 36.5 - 37.2 C |
| Coat condition | good |
| diabetes | negative |
| BUN | 1.2 - 2.3 (IU/L) |
| heart rate | 315 - 476 (beats/minute) |
| behavior | normal |

### [eighth embodiment]

### Two dimensional image analysis

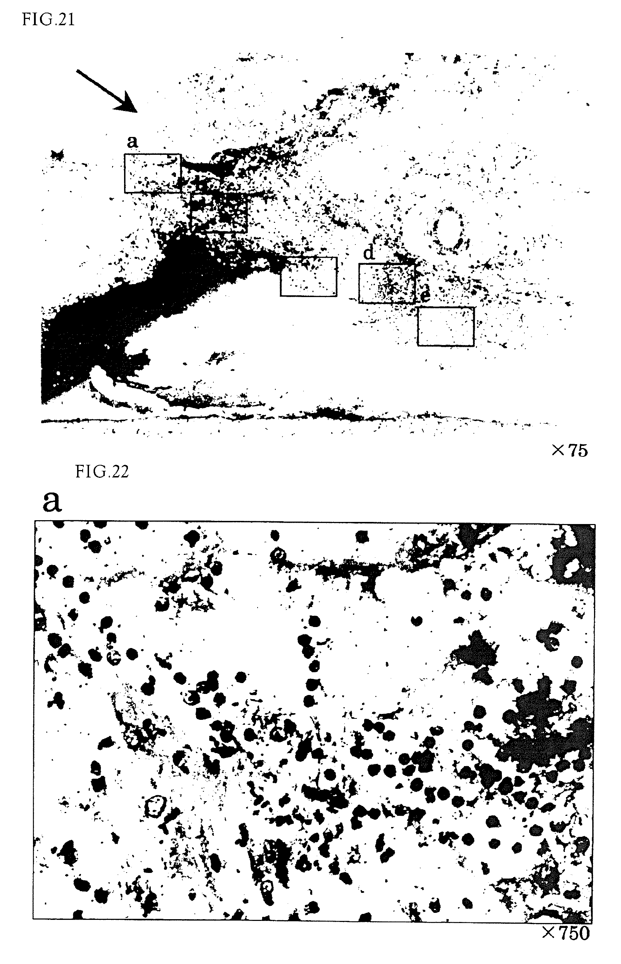

After the seventh embodiment was conducted, histological stains of the surgery site and the indwelling needle punctured sites of the aorta and vena cava as well as the stem cell DNA micro-autoradiograph images of [2-14C] thymidine were prepared. The result indicates that the recovery from the damage was significantly active with respect to the experimental result using the stainless steel indwelling needle (Figs. 21 - 30). Fig. 21 shows a histologic photograph of three days after the punctuation with multi-purpose stainless steel needle of 14 gauge at the downward portion of right inferior vena cava of a rat. There is an arrow at the left top in the figure, a wide imprint of the puncture of the stainless steel needle from the direction pointed to obliquely right bottom. Inwardly from the perithelium of the inferior vena cava, rectangular frames in the order of a, b, c, d, and e are marked on the imprint. These sections are enlarged in Figs. 22 to 26 as enlarged view.

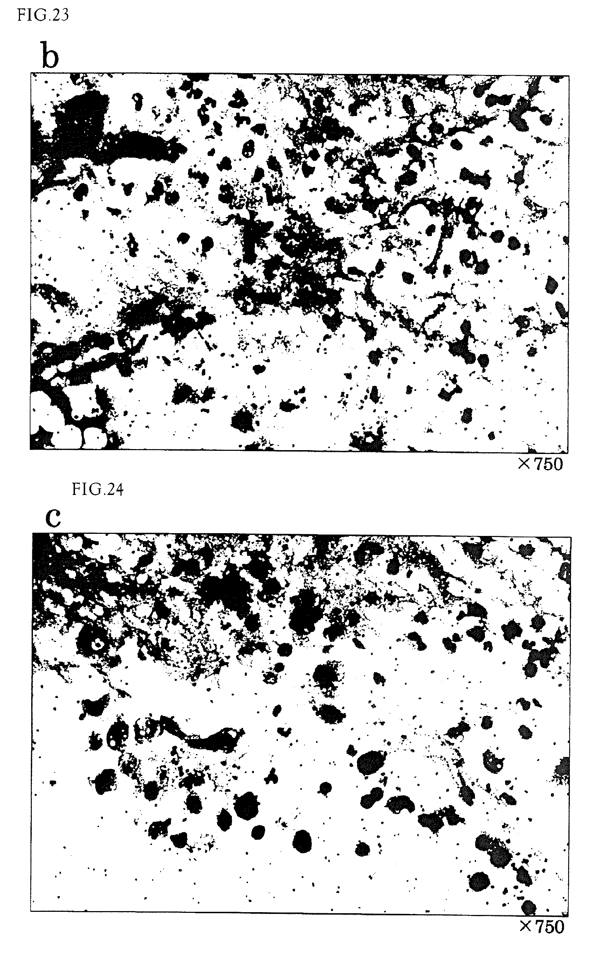

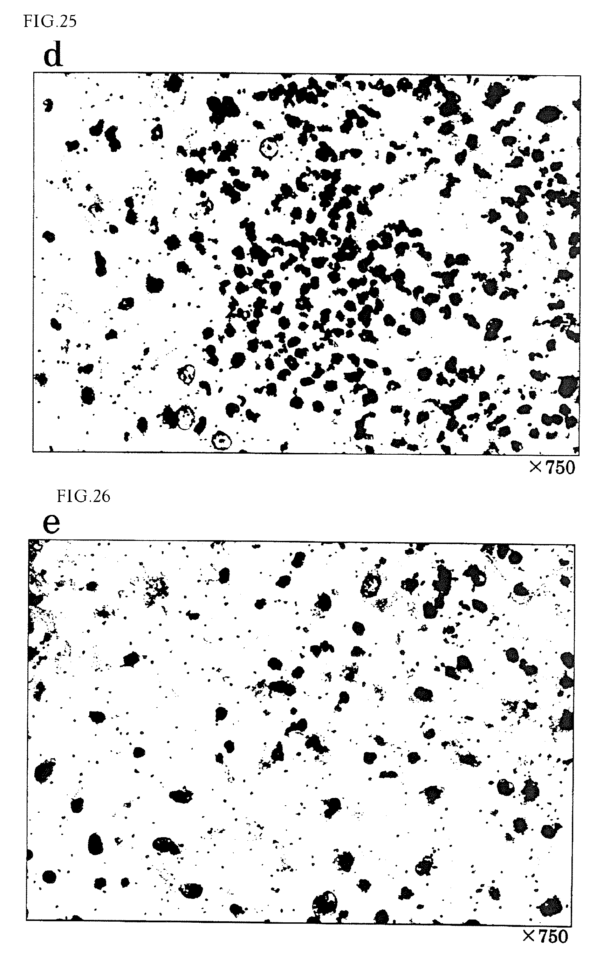

Fig. 22 shows a number of disseminated basophiles, which secrete actively protease so as to heal the damage. The silver particles indicative of cell restoration are not distributed to nuclei. Fig. 23 shows disseminated eosinophiles. However the regeneration of cells is not recognized. Fig. 24 shows an enlarged view of blood vessel endothelium, which supports the bloodstream in the vena cava, the recovery of endothelium is still insufficient, the cell membrane of one layer is not observed, and only the suspended eosinophiles are dispersed. Fig. 25 shows a group of neutrophiles. This figure shows that the phagocytosis is active. Fig. 26 shows only the floated eosinophiles, and the regeneration is not still observed.

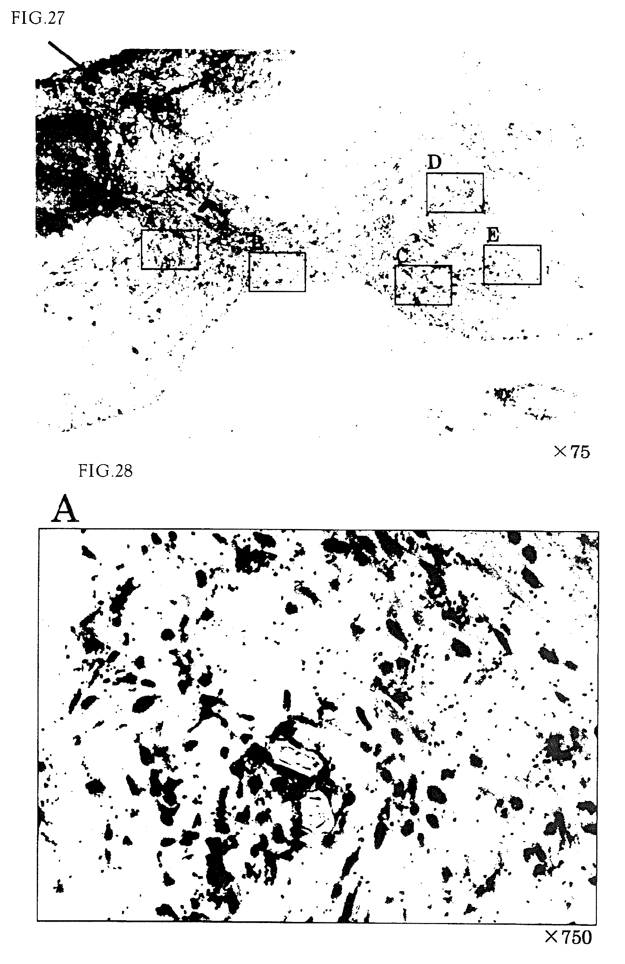

Fig. 27 to 32 show histological photographs three days after the puncture by the ceramic hollow needle of 14G in accordance with the present invention. The arrow at the left top of Fig. 27 indicates the puncture of the hollow needle of the present invention in that direction. The significant difference from the case of the stainless steel needle is that the imprint of the puncture was extremely narrowed. More specifically, there already exists wider recovered part. The sections labeled as A, B, C, D, and E with a rectangular frame are enlarged in Figs. 28 to 32.

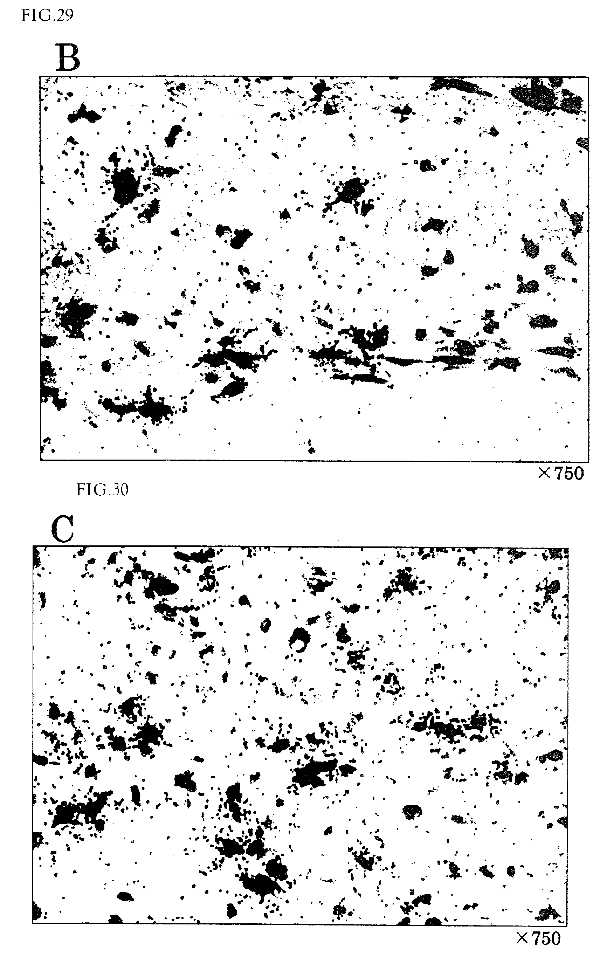

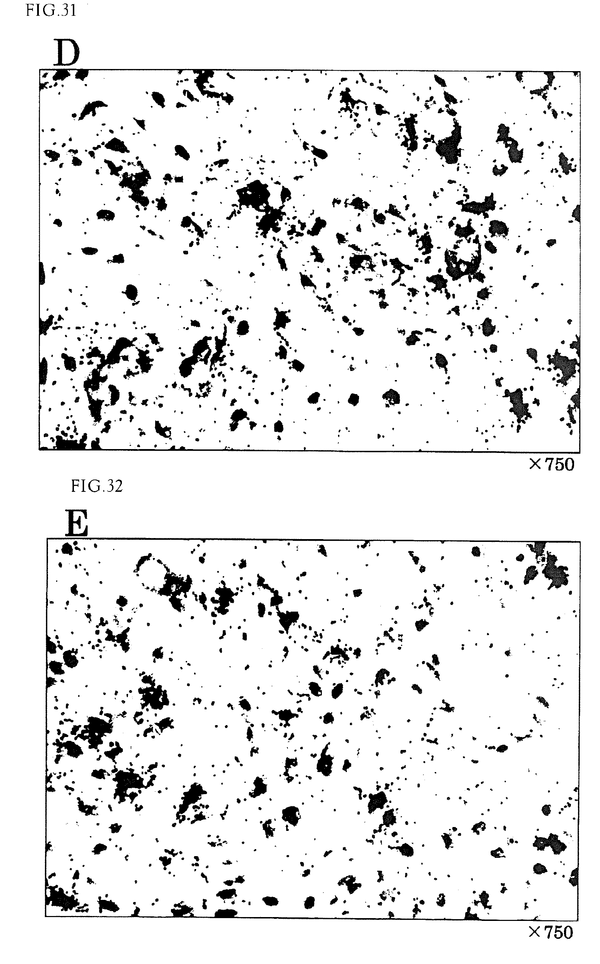

Fig. 28 is an enlarged view of the right side of the puncture imprint in the vicinity of perithelium. The mass of cells in the center of the histologic image is the center of muscular stem cells which appear at the early stage of blood vessel regeneration, and is called as germinative center. Small cell nuclei like planets are disseminated around the center, and silver particles are promptly recognized in the nuclei indicating the DNA synthesis for the regeneration. Fig. 29 shows the endothelium of the blood vessel facing to the blood stream in the vena cava, indicating that the regeneration was completed three days after the punctuation. A number of silver particles is observed in the nuclei not only in the endothelial cells but also in the deep cell layer. Fig. 30 shows the left side of the damaged lesion three days after the punctuation with a ceramic hollow needle. There are shown a group of silver particles in a number of germinal centers and the periphery thereof, indicating the uptake of [2-14C] thymidine into the nuclei as the indication of cell mitosis therearound. In Fig. 31 and Fig. 32 silver particles are observed in a number of germinative centers and peripheric cell nuclei, however no leucocyte is observed. The phagocyte indicative of the onset of inflammation and the imprint of the damage are no longer present. These histological photographs proves the advantage of the ceramic hollow needle in accordance with the present invention over the stainless steel hollow needle.

As can be appreciated from the foregoing description, zirconia ceramic needle gives less damage to the tissue at the punctuation site of hollow needle, when compared with a stainless steel needle.

### Availability in the industry

The hollow needle made of a ceramic in accordance with the present invention does not impart significant tissue damage at the needle punctuation site, and is allowed to use as the injection needle and indwelling needle, in particular the injection needle and indwelling needle for the long time blood collection and long time blood infusion. A typical example of the long time blood collection and infusion is the dialysis. Since the tissue damage at the needle punctuation site is much less, the hollow needle and the indwelling needle using the hollow needle in accordance with the present invention may contribute in the medical and care field.

### Brief description of the drawings

Fig. 1 is the tissue after the punctuation of a stainless steel hollow needle to the inferior vena cava of a rat;
Fig. 2 is an enlarged view of the section labeled by a in Fig. 1;
Fig. 3 is an enlarged view of the section labeled by b in Fig. 1;
Fig. 4 is an enlarged view of the section labeled by c in Fig. 1;
Fig. 5 is an enlarged view of the section labeled by d in Fig. 1;
Fig. 6 is an enlarged view of the section labeled by e in Fig. 1;
Fig. 7 is an enlarged view of the section labeled by f in Fig. 1;
Fig. 8 is an enlarged view of the section labeled by g in Fig. 1;
Fig. 9 shows the tissue after the punctuation of a zirconia hollow needle in the inferior vena cava of a rat;
Fig. 10 is an enlarged view of the section labeled by A in Fig. 9;
Fig. 11 is an enlarged view of the section labeled by B in Fig. 9;
Fig. 12 is an enlarged view of the section labeled by C in Fig. 9;
Fig. 13 is an enlarged view of the section labeled by D in Fig. 9;
Fig. 14 is an enlarged view of the section labeled by E in Fig. 9;
Fig. 15 is an enlarged view of the section labeled by F in Fig. 9;
Fig. 16 is an enlarged view of the section labeled by G in Fig. 9;
Fig. 17 is a side view of a hollow needle installed to a straight fine tube;
Fig. 18 is a side view of a hollow needle outer tube;
Fig. 19 is a side view of a connection tube;
Fig. 20 is a schematic representation of an indwelling needle;
Fig. 21 shows the tissue after the punctuation of a stainless steel indwelling needle to the inferior vena cava of a rat;
Fig. 22 is an enlarged view of the section labeled by a in Fig. 21;
Fig. 23 is an enlarged view of the section labeled by b in Fig. 21;
Fig. 24 is an enlarged view of the section labeled by c in Fig. 21;
Fig. 25 is an enlarged view of the section labeled by d in Fig. 21;
Fig. 26 is an enlarged view of the section labeled by e in Fig. 21;
Fig. 27 shows the tissue after the punctuation of a zirconia indwelling needle to the inferior vena cava of a rat;
Fig. 28 is an enlarged view of the section labeled by A in Fig. 27;
Fig. 29 is an enlarged view of the section labeled by B in Fig. 27;
Fig. 30 is an enlarged view of the section labeled by C in Fig. 27;
Fig. 31 is an enlarged view of the section labeled by D in Fig. 27;
Fig. 32 is an enlarged view of the section labeled by E in Fig. 27;

### Reference numerals

A, B, C, D, E, F, G symbol indicating enlarged sections of tissue
a, b, c, d, e, f, g symbol indicating enlarged sections of tissue
1 hollow needle with the straight fine tube
2 straight fine tube
3 hollow needle
4 Millipore filter section
5 head
11 hollow needle outer tube
12 center tube
14 receiving tube
21 connection tube
22 main connection tube
23 connection ring
24 needle ring
26 clip
31 indwelling needle

## Claims

1. A hollow needle made of a ceramic, having the outer diameter of 1 to 5 mm.

2. A hollow needle in accordance with claim 1, in which the wall thickness is from 0.3 mm to 1.8 mm.

3. A hollow needle in accordance with claim 1 or claim 2, manufactured by providing a heat resistant wire used as core, covering the outside of the core with paste of ceramic fine powder and burning, then removing said wire after burning.

4. A hollow needle in accordance with any one of claim 1 to 3, in which
the ceramic is the fine powder of zirconium or zirconia.

5. A hollow needle in accordance with any one of claim 1 to 4, in which
non punctuating end of the hollow needle is inserted into a straight fine tube having a thick wall, made of a chemical resistant, corrosion resistant resin.

6. A hollow needle in accordance with claim 5, further comprising
non-woven cotton at the other end of said straight fine tube.

7. An indwelling needle using the hollow needle in accordance with any one of claim 1 to 6.

8. An indwelling needle, made by installing the non punctuating end of a hollow needle in accordance with claim 5 or claim 6 into a thick straight fine tube made of a chemical resistant, corrosion resistant resin, and inserting said hollow needle made of a ceramic into a hollow needle outer tube made of a chemical resistant, corrosion resistant resin.

9. An indwelling needle in accordance with claim 8, in which
said hollow needle outer tube has the portion for inserting a hollow needle composed of a thick center tube made of a chemical resistant, corrosion resistant resin, and has the portion for inserting a thick straight fine tube made of a chemical resistant, corrosion resistant resin installed to the non punctuating end of the hollow needle, composed of a thinner receiving tube made of a chemical resistant, corrosion resistant resin.

10. An indwelling needle in accordance with claim 8 or claim 9, said indwelling needle being prepared by inserting a mold into a thin tube made of a chemical resistant, corrosion resistant resin, and entirely shrinking to integrate a thick and fine center tube with a thin receiving tube.

11. An indwelling needle in accordance with any one of claim 8 to claim 10, in which a flexible connection tube for connecting to an external extension tube is provided to said hollow needle outer tube.

12. An indwelling needle in accordance with claim 11, in which said connection tube is provided on said receiving tube.

13. An indwelling needle in accordance with claim 11 or claim 12, in which a clip is provided on said connecting tube for temporarily stop the fluid flowing through the connecting tube.

14. An indwelling needle in accordance with any one of claim 11 to claim 13, in which a needle ring is provided at the hollow needle end of said connecting tube, and a connection ring at the other end.

15. An indwelling needle in accordance with any one of claim 5, claim 8, claim 9, and claim 10, in which said chemical resistant, corrosion resistant resin is a fluorocarbon resin.

16. An indwelling needle in accordance with any one of claim 11 to claim 14, in which said connecting tube is made of silicone.
